# EUROPEAN PATENT SPECIFICATION

(11) **EP 0 365 562 B1**
(45) Date of publication and mention of the grant of the patent: **09.04.1997**
(21) Application number: 88905545.5
(22) Date of filing: 02.06.1988
(51) Int. Cl.: C12N 15/00, A01K 67/02

(54) **BOVINE NUCLEAR TRANSPLANTATION**
TRANSPLANTATION VON RINDERZELLKERNEN
TRANSPLANTATION NUCLEAIRE BOVINE

(30) Priority: 05.06.1987 US 58904
(43) Date of publication of application: 02.05.1990
(73) Proprietor: ABS Global, Inc., New York, N.Y. 10169 (US)
(72) Inventor: MASSEY, Joseph, M., College Station, TX 77840 (US); WILLADSEN, Steen, M., Calgary, Alberta T2M 4N3 (CA)
(74) Representative: Bentham, Stephen
(86) International application number: US8801906
(87) International publication number: WO8809816

(56) References cited:
- US-A- 4 664 097
- Journal of Animal Science, vol. 64, no. 2, February 1987; J.M. Robl et al.: "Nuclear transplantation in bovine embryos", pages 642-647
- Theriogenology, vol. 25, no. 1, January 1986; J.M. Robl et al.: "Nuclear transplantation in bovine embryos", page 189
- Nature, vol. 320, 6 March 1986; S.M. Willadsen: "Nuclear transplantation in sheep embryos", pages 63-65
- Biology of Reproduction, vol. 37, no. 4, 1987; R.S. Prather et al.: "Nuclear transplantation in the bovine embryo: assessment of donor nuclei and recipient oocyte", pages 859-866

## Description

Nuclear transfer involves the transplantation of living nuclei from typically embryonic cells to unfertilized eggs. The early research on vertebrates was performed in amphibians. The embryonic frog blastomere cells were separated and the nuclear material was introduced into frogs' eggs which had been enucleated. See Transplantation of Living Nuclei From Blastula Cells into Enucleated Frogs Eggs, R. Briggs and T.J. King, Proceedings of the National Academy of Sciences, Volume 38, pages 455 - 463, 1952. Further experimentation was performed on amphibians and amphibian eggs to determine if nuclear material from adult frog somatic or germinal cells could be transplanted to eggs and develop into a normal larva. Development and Chromosomal Constitution of Nuclear-Transplants Derived from Male Germ Cells, M. A. DiBerardino and N. Hoffner, Journal of Experimental Zoology, Volume 176, pages 61 - 72, 1971. There is a great degree of uncertainty as to when the genetic material of a cell can no longer be reprogrammed, which limits the stage of development of donor nuclear material.

Transplantation of nuclear material in mammals has proved very difficult to achieve which is in part due to the microsurgical techniques on mammalian embryos and eggs. The microsurgical techniques can be destructive to delicate cell structure which damages the cell material used in the later stage of the transplantation procedure. An alternative procedure is to deliver the nuclear material to a recipient egg by fusion of an intact cell or a karyoplast consisting of a nucleus surrounded by a piece of plasma membrane to the egg. The manipulation to isolate a karyoplast is performed in the presence of cytochalasin B. Methods and Success of Nuclear Transplantation in Mammals, A. McLaren Nature, Volume 109, June 21, 1984. Also, once the nuclear material has been transplanted to a recipient a mammalian egg there is fusion of the cellular material to produce a new viable embryo. The fusion can be aided or induced with virus or electro-field induced. However, the conditions for fusion are not predictable. Electric Field-Induced Cell-to-Cell Fusion, U. Zimmermann and J. Vienke, Journal of Membrane Biology, Volume 67, page 165 - 182 (1982).

Nuclear transplantation in higher mammals has been attempted. Successful nuclear transplantation and cell fusion was achieved for sheep embryos when individual blastomeres from 8 and 16 cell embryos were used as the nuclear donors into enucleated or nucleated halves of unfertilized eggs. Nuclear Transplantation in Sheep Embryos, S.M. Willadsen, Nature, Volume 320, pages 63 - 65, March 1986. Nuclear transplantation has been attempted in bovine embryos, however, the embryos developed only 43 days out of a nine month typical gestation period. Nuclear Transplantation in Bovine, J. M. Robl, R. Prather, W. Eyestone, F. Barnes, D. Northey, B. Gilligan, and N. L. First, Theriogenology, Volume 25, No. 1, January 1986. Successful nuclear transplantation and embryo development in higher mammals has great implications in breeding.

Selective cattle breeding involves the selection of the desired bull as the sperm donor and the desired female for the egg donor. Typically the female was artificially inseminated with the bull semen in a selective breeding program. The female may be superovulated to produce several eggs and therefor several embryos. The embryos may then be transferred to recipient cows for gestation.

The present invention provides a process of in vitro nuclear transplantation including the following steps:
(a) removing a portion of the ooplasm from a recipient oocyte collected from a donor cow;
(b) separating into single donor cells for nuclear transplantation the cells of a bovine embryo of from equal to or greater than the 32 cell stage;
(c) inserting a donor cell into the perivitelline space of the recipient oocyte; and
(d) electrofusing the oocyte fragment and donor cell to form an embryo suitable for culturing to provide an embryo transferable into a recipient cow for gestation.

The process of the invention may be used to produce a viable bovine embryo.

This method may be used to produce a relatively large number of identical bovine embryos that can be transferred to surrogate female cows for gestation. The process involves the separation of a donor embryo into single cells which were transplanted into nucleated or enucleated oocytes for further development. The donor embryo can be from a 32 cell embryo to at least a compacted morula stage embryo (approximately 64 cells). The embryos can be removed non-surgically which is a more economical method than surgical removal. For commercial breeding application the embryos used as nuclear donors are selected from cows that have been inseminated with the selected bull semen to produce the desired cross or an embryo developed from a prior nuclear transfer. However, this process may be used with any bovine multicellular embryo. Also, good results have been obtained with previously deep frozen embryos.

The stage of the donor embryo at the 32 cell to compacted morula stage of development makes the process attractive from an economical standpoint because of the increased number of donor nuclear cells as well as the lack of surgical technique necessary to remove the embryo initially. The process, whether or not a surgical procedure is used to remove embryos, enables one to prepare embryo clones with a good success rate in viability. Although there were some losses in viability of the embryos during the early stage of development, this process will produce multiple calves with identical genetic makeup.

Also, embryos that were developed through this procedure have in turn been used as nuclear donor cells for subsequent recipient eggs. This produces viable cattle embryos by transplantation of nuclear material from embryos which are themselves the products of nuclear transplantation.

### Preferred Process

The production of a viable embryo using the process of the invention constitutes several steps which generally includes isolating the recipient oocytes from the donor cows, recovering embryos from donor cows, transferring the nuclear material from the embryo to the recipient oocytes, fusing the oocyte fragment and the donor cells to form an embryo, and culturing the embryo before transferring into a recipient cow for gestation. The recipient oocytes are collected from cows which have been induced to ovulate at a predicted time with prostaglandin F₂ alpha (PGF₂α). Human chorionic gonadotropin (hCG) can also be administered to further predict the ovulation time. The oocytes were collected approximately 39 hours after hCG or 87 hours after PGF₂ α administration. Approximately 4,000 I.U. of hCG were administered. If desired, the cow can be superovulated to produce multiple eggs by administering FSH hormone. The oocytes were collected surgically approximately 39 hours after hCG administration. This is approximately 10 to 14 hours after ovulation.

The zona pellucida of the egg was cleaned of all cells and debris with a cleaning pipette. The eggs were placed in a solution of Dulbecco's Phosphate Buffed Saline (PBS). After cleaning the eggs were placed on a micromanipulation stage. Using a fine glass needle, a slit was made in the zona pellucida directly over the polar body traversing the perivitelline space such that the zona pellucida is opened slightly more than 180° along its equator with the polar body near the middle of the slit. If the polar body was not located, a slit of the same dimension was made in the part of the zona pellucida which spans the widest portion of the perivitelline space.

After the recipient oocytes have been slit, they were placed in PBS containing Cytochalasin B (5 micrograms per/ml; Sigma) for at least half an hour before further processing.

The nucleus donor embryo is typically collected from a cow which has been artificially inseminated. However the donor embryo may be any cow embryo from the 32 cell to compacted morula stage of development. For embryo donors from the 32 cell to compacted morula stage, nonsurgical procedures are useable to flush the embryo from the cow uterus. The donor embryos are collected after four and half to six days after behavioral estrus is observed. The embryos are examined after collection to determine the stage of development. In some cases the embryo nuclear donor was from deep frozen embryos. Typically 32 cell embryos are collected on day five. Also late morulae and early blastocysts collected on day six have been used.

The zona pellucida of the nucleus donor embryo was slit with a fine glass needle 3/4 or more around the equator. The embryo was coaxed out of the zona pellucida with the tip of the needle and transferred to a petri dish with fresh PBS solution. The embryo was separated into blastomere cells by repeated suction in a micropipette. An alternative procedure is to remove the individual cells from the embryo by drawing the cells one at a time into a micropipette with an inner diameter approximately the same size as the cells.

Returning to the oocytes which have been previously slit, if the polar body was clearly visible, the polar body and an adjacent part of the ooplasm corresponding to between 1/4 and 1/8 of the total volume of the cell was sucked into a micropipette. In oocytes with no visible polar body, about 1/2 of the ooplasm was removed by suction with a micropipette. The ooplasm which was removed can be inserted into an evacuated zona pellucida. The foreign zona pellucida can be obtained from an egg that is not chosen as a recipient oocyte. Thus, one egg can yield two recipient oocytes if a foreign zona pellucida is used.

An alternative procedure in preparing the egg utilizes a micropipette to pierce the zona pellucida. A micropipette was inserted in the egg- and about half of ooplasm was aspirated. The zona pellucida encloses the remaining ooplasm of the donor egg and the ooplasm removed with the micropipette was placed in a recipient zona pellucida.

The enucleated half of the egg, the half without the polar body, is presumed to fuse with the nuclear material that comes from the separated embryo cells. However, sometimes it is not possible to identify the polar body so it was difficult to determine where the nuclear material lies. It has been postulated that an egg with nuclear material should not be viable with additional nuclear material from the embryo cell. There is sometimes more than a 50% survival rate when one egg is divided and nuclear material transplanted into both halves. This supports the premise that possibly the nuclear material was broken up to a degree that it is not viable. However, other theories could support the simultaneous viable development of both egg halves.

The disassociated blastomeres of the donor embryo were transferred to the halved recipient oocytes. A blastomere was transferred to the perivitelline space and brought in close contact with the oocyte fragment. The blastomere/oocyte fragments were then ready for fusion. The preferred method of fusion is electrofusion.

The embryos were placed in PBS at room temperature for one hour before electrofusion. The zonae pellucidae containing blastomere and egg halves were transferred to a petri dish containing fusion medium of 0.3M Mannitol, 0.1 mM MgSO₄, 0.05 mM CaCl₂ in distilled water or Zimmerman cell fusion media for approximately 25-30 minutes.

The blastomere/oocytes were transferred in the fusion medium to the chamber of an electrofusion apparatus (Zimmermann; GCA, Chicago). With the Zimmerman electrofusion apparatus the two electrical poles are wires which are parallel to each other in the center of the plate. The oocyte with the blastomere are lined up at right angles to either wire such that the oocyte touches the wire and the blastomere portion is opposite to the wire. The blastomere/oocytes are exposed to the following fusion conditions; cell alignment (600 kHz, 6V for 10 seconds), followed by 3 fusion pulses of 15 V with a pulse duration of 50.0 microseconds at 0.1 second interval. After the fusion pulses, the alignment voltage was reduced over one minute from 6 V to 0 V. All embryos were transferred to PBS and incubated at 37° C for one hour. The embryos are then examined to determine in which fusion had occurred or is in progress.

A semi-solid medium such as agar can be used to embed the embryos prior to transfer to sheep oviducts. Several embryos can be embedded in one section of agar. The agar chip allows for normal development to occur in the ewe's oviduct even though the zonae pellucidae are not intact.

An agar solution was prepared with 0.6 g agar dissolved in 60 ml of 0.9% NaCl in distilled water by boiling. The agar solution was set aside to cool. The embryos were transferred to a PBS solution with 20% sheep or calf serum. When the agar solution has cooled to 30 to 35° C the embryos are picked up in a small amount of PBS medium with a micropipette. Five to 10 ml of agar solution was poured into an empty petri dish and the embryos are transferred into the agar using the pipette. The agar containing the embryo was then placed in a petri dish containing PBS. The embryo in the agar is expelled from the pipette shaped as a small cylinder which is cut short as possible.

A second embedding takes place using a 0.6 gm agar dissolved in 50 ml of 0.9% NaCl in distilled water by boiling. The agar solution was cooled to 30 to 35° C. The first embedded embryos were completely covered. The second embedding with agar formed a chip not longer than 2.5 to 3 mm.

The agar embedded embryos were transferred into ligated oviducts of a ewe for culture. Four to six days later the oviduct ewe was flushed with PBS solution which is inspected for the chip with the embedded embryo. The chip was cut open so that the cavity around the embryo is open and the embryo is dislodged with glass needles. After release from the agar chip the embryos were placed in fresh PBS at room temperature until they can be transferred to recipient cows in a conventional way.

The nucleus donor embryo used in this procedure can be a embryo which was produced from the nuclear transplantation procedure of this invention. This serial use of the nuclear material involves the use of nucleus donor embryo prepared following the steps of the above described procedure. The embryo recovered from the sheep is at a stage of development which can be used as a nucleus donor. The embryo cells are separated and fused with recipient oocytes as described above. The use of nuclear donor embryos prepared by nuclear transplantation can be performed serially such that nuclear material can be from several generations of embryonic material prepared by the procedures described herein.

The embryos recovered from the sheep may be deep frozen. At a later time the embryos are thawed and used in the procedure as described herein.

The following Tables I and II Examples give the results of the nuclear transplantation of bovine embryo cells to oocytes as described by this procedure. Table I is a summary of first generation transfers in which the embryo used as the nucleus donor was from an artificially inseminated cow. Table II is a summary of second generation transfer in which the embryo used as the nucleus donor was from a first generation embryo developed by the nuclear transplantation process.

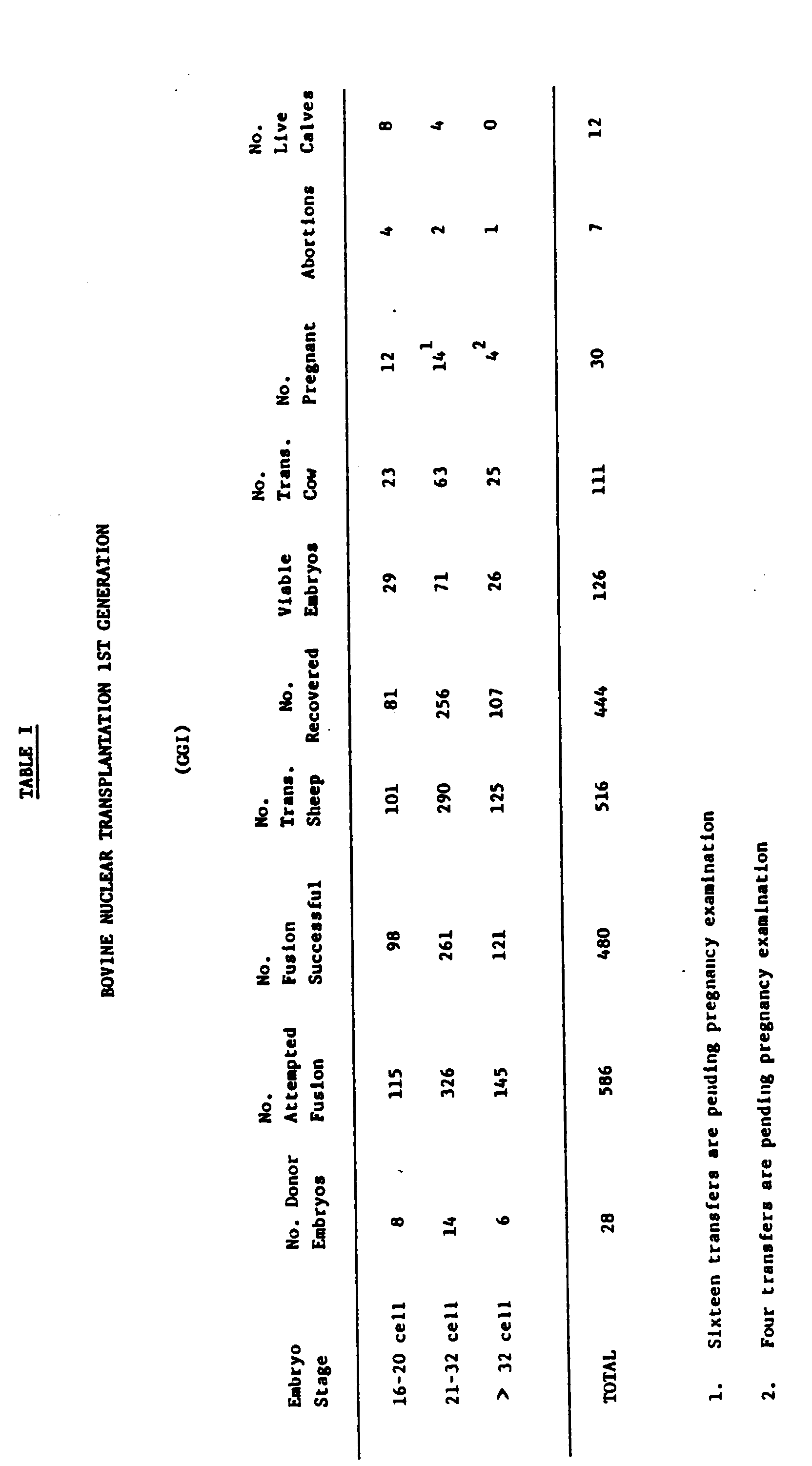

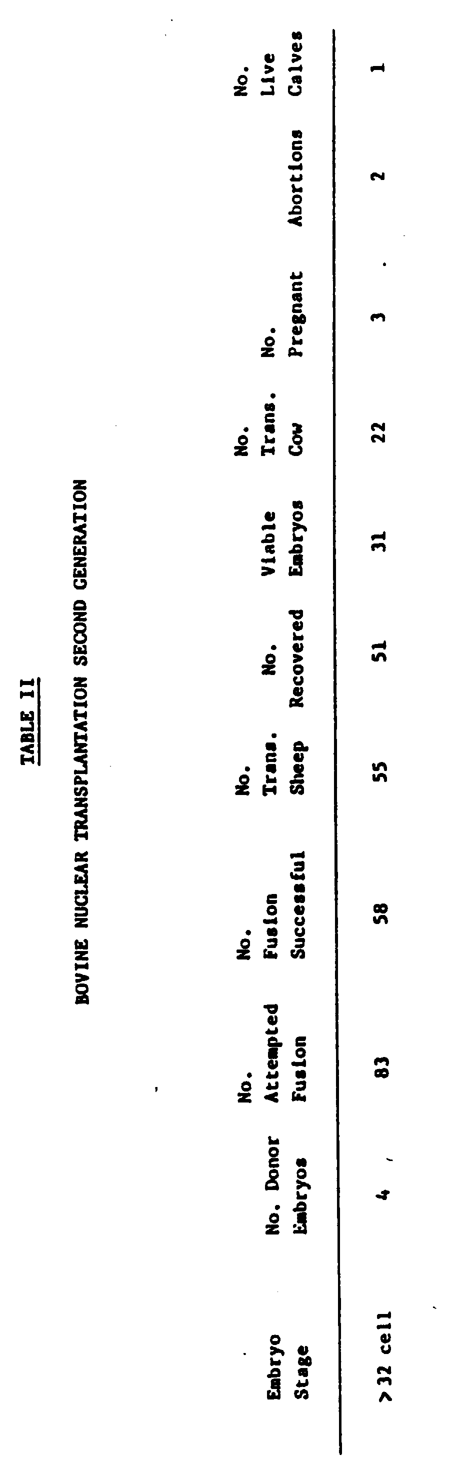

As shown in Table I, 16 cell stage embryos and higher were used. The highest cell stage tested was compacted morula of about 64 cells. The lower cell stage embryo nucleus donors were traditionally thought to be more suitable for transfer of nuclear material. However, Table I shows a good fusion and viability rate of the higher cell stage embryo compared to the lower cell stage.

The number of successful fusions are noted on both Table I and Table II. This figure was calculated based on visual observation of the embryos under a microscope after electrofusion. The number of successful fusions was rated by visual observation. Some of the embryos which did not pass the visual test for fusion were nevertheless transferred to the sheep for further culture.

Not all the embryos were carried through all the stages of the process as shown in the Tables. Live calves were born from the process of this invention demonstrating the success of the methods described.

Table II shows the use of second generation embryos, those developed by this invention, in subsequent nuclear transplantation. The embryos recovered from the sheep are used as nuclear material donors. Consequently, a descending number of generations can be cloned using the same genetic material using the steps of this transplantation process.

## Claims (Claims for the following Contracting State(s): AT, BE, IT, LU, SE)

1. Process of in vitro nuclear transplantation including the following steps:
(a) removing a portion of the ooplasm from a recipient oocyte collected from a donor cow;
(b) separating into single donor cells for nuclear transplantation the cells of a bovine embryo of from equal to or greater than the 32 cell stage;
(c) inserting a donor cell into the perivitelline space of the recipient oocyte; and
(d) electrofusing the oocyte fragment and donor cell to form an embryo suitable for culturing to provide an embryo transferable into a recipient cow for gestation.

2. A process according to claim 1 in which the donor cells in step (b) are from an embryo of from the 32 cell stage to compacted morula stage.

3. A process according to claim 1 or 2 in which the donor cells in step (b) are from an embryo recovered by flushing the embryo from the uterus of an inseminated donor cow by a non-surgical procedure.

4. A process according to claim 1, 2 or 3 in which the recipient oocyte of step (a) has been collected approximately 10 to 14 hours post-ovulation.

5. A process according to claim 4 in which the oocyte has been collected approximately 39 hours after administering human chorionic gonadotropin or 87 hours after administering prostaglandin F₂α to the donor cow.

6. A process according to any one of the preceding claims wherein the donor cells in step (b) are from an embryo which is deep frozen for use later and thawed before such use.

7. A process according to any one of the preceding claims wherein in step (a) the portion of the ooplasm removed is adjacent to and includes the polar body.

8. A process according to claim 7 wherein in step (a) the portion of ooplasm removed is from a quarter to a eighth of the total volume of the cell.

9. A process according to any one of claims 1 to 7, wherein in step (a) about half of the ooplasm is removed from the recipient oocyte and inserted into a foreign zona pellucida, said foreign zona pellucida having been prepared to receive the ooplasm by removal from an oocyte, and which comprises the steps (b) to (d) using the foreign zona pellucida with the inserted ooplasm as recipient oocyte.

10. A process according to any one of the preceding claims wherein the donor cells are from an embryo from an inseminated donor cow or from a prior nuclear transplantation as defined by steps (a) to (d) in claim 1.

## Claims (Claims for the following Contracting State(s): DE, GB, NL, FR)

1. Process of in vitro nuclear transplantation including the following steps:
(a) removing a portion of the ooplasm from a recipient oocyte collected from a donor cow;
(b) separating into single donor cells for nuclear transplantation the cells of a bovine embryo of greater than the 32 cell stage;
(c) inserting a donor cell into the perivitelline space of the recipient oocyte; and
(d) electrofusing the oocyte fragment and donor cell to form an embryo suitable for culturing to provide an embryo transferable into a recipient cow for gestation.

2. A process according to claim 1 in which the donor cells in step (b) are from an embryo of from the 32 cell stage to compacted morula stage.

3. A process according to claim 1 or 2 in which the donor cells in step (b) are from an embryo recovered by flushing the embryo from the uterus of an inseminated donor cow by a non-surgical procedure.

4. A process according to claim 1, 2 or 3 in which the recipient oocyte of step (a) has been collected approximately 10 to 14 hours post-ovulation.

5. A process according to claim 4 in which the oocyte has been collected approximately 39 hours after administering human chorionic gonadotropin or 87 hours after administering prostaglandin F₂α to the donor cow.

6. A process according to any one of the preceding claims wherein the donor cells in step (b) are from an embryo which is deep frozen for use later and thawed before such use.

7. A process according to any one of the preceding claims wherein in step (a) the portion of the ooplasm removed is adjacent to and includes the polar body.

8. A process according to claim 7 wherein in step (a) the portion of ooplasm removed is from a quarter to an eighth of the total volume of the cell.

9. A process according to any one of claims 1 to 7, wherein in step (a) about half of the ooplasm is removed from the recipient oocyte and inserted into a foreign zona pellucida, said foreign zona pellucida having been prepared to receive the ooplasm by removal from an oocyte, and which comprises the steps (b) to (d) using the foreign zona pellucida with the inserted ooplasm as recipient oocyte.

10. A process according to any one of the preceding claims wherein the donor cells are from an embryo from an inseminated donor cow or from a prior nuclear transplantation as defined by steps (a) to (d) in claim 1.

## Patentansprüche (Patentansprüche für folgende(n) Vertragsstaat(en): AT, BE, IT, LU, SE)

1. Verfahren zur in vitro Zellkerntransplantation, umfassend die folgenden Schritte:
(a) Entfernen eines Teils des Eiplasmas aus einer Empfänger-Eizelle, die einer Spenderkuh entnommen wurde;
(b) Trennen in einzelne Spenderzellen zur Zellkerntransplantation der Zellen eines Rinderembryos von gleich oder größer dem 32. Zellstadium;
(c) Einbringen einer Spenderzelle in den perivitellinen Raum der Empfänger-Eizelle; und
(d) Elektroverschmelzen des Eizell-Fragments mit der Spenderzelle zum Erhalt eines züchtbaren Embryos, um einen in eine Empfänger-Kuh zur Gestation einpflanzbaren Embryo zur Verfügung zu stellen.

2. Verfahren nach Anspruch 1, bei welchem die Spenderzellen in Schritt (b) von einem Embryo vom 32. Zellstadium bis zum verdichteten Morulastadium stammen.

3. Verfahren nach Anspruch 1 oder 2, bei welchem die Spenderzellen in Schritt (b) von einem durch Spülen des Embryos aus dem Uterus einer befruchteten Spender-Kuh mittels eines nicht-operativen Verfahrens rückgewonnenen Embryo stammen.

4. Verfahren nach Anspruch 1, 2 oder 3, bei welchem die Empfänger-Eizelle aus Schritt (a) etwa 10 bis 14 Stunden nach der Ovulation entnommen wurde.

5. Verfahren nach Anspruch 4, bei welchem die Eizelle etwa 39 Stunden nach Verabreichung von chorialem Human-Gonadotropin oder 87 Stunden nach Verabreichung von Prostaglandin F₂α/San die Spender-Kuh entnommen wurde.

6. Verfahren nach einem der vorangegangenen Ansprüche, wobei die Spenderzellen in Schritt (b) von einem Embryo stammen, der zur späteren Verwendung tiefgefroren und vor dieser Verwendung aufgetaut wurde.

7. Verfahren nach einem der vorangegangenen Ansprüche, wobei in Schritt (a) der Anteil des entfernten Eiplasmas der an das Polkörperchen angrenzende und dieses enthaltende ist.

8. Verfahren nach Anspruch 7, wobei in Schritt (a) der Anteil des entfernten Eiplasmas von einem Viertel bis zu einem Achtel des Gesamtvolumens der Zelle beträgt.

9. Verfahren nach einem der Ansprüche 1 bis 7, wobei in Schritt (a) etwa die Hälfte des Eiplasmas aus der Empfänger-Eizelle entfernt und in eine Fremdeihülle eingefügt wird, wobei diese Fremdeihülle zur Aufnahme des Eiplasmas durch Entfernung aus einer Eizelle vorbereitet worden war, und welches die Schritte (b) bis (d) unter Verwendung der Fremdeihülle mit dem eingefügten Eiplasma als Empfänger-Eizelle umfaßt.

10. Verfahren nach einem der vorangegangenen Ansprüche, wobei die Spenderzellen von einem Embryo einer befruchteten Spender-Kuh oder einer früheren Zellkerntransplantation, wie durch Schritte (a) bis (d) in Anspruch 1 definiert, stammen.

## Patentansprüche (Patentansprüche für folgende(n) Vertragsstaat(en): DE, GB, NL, FR)

1. Verfahren zur in vitro Zellkerntransplantation, umfassend die folgenden Schritte:
(a) Entfernen eines Teils des Eiplasmas aus einer Empfänger-Eizelle, die einer Spenderkuh entnommen wurde;
(b) Trennen in einzelne Spenderzellen zur Zellkerntransplantation der Zellen eines Rinderembryos von größer als dem 32. Zellstadium;
(c) Einbringen einer Spenderzelle in den perivitellinen Raum der Empfänger-Eizelle; und
(d) Elektroverschmelzen des Eizell-Fragments mit der Spenderzelle zum Erhalt eines züchtbaren Embryos. um einen in eine Empfänger-Kuh zur Gestation einpflanzbaren Embryo zur Verfügung zu stellen.

2. Verfahren nach Anspruch 1, bei welchem die Spenderzellen in Schritt (b) von einem Embryo vom 32. Zellstadium bis zum verdichteten Morulastadium stammen.

3. Verfahren nach Anspruch 1 oder 2, bei welchem die Spenderzellen in Schritt (b) von einem durch Spülen des Embryos aus dem Uterus einer befruchteten Spender-Kuh mittels eines nicht-operativen Verfahrens rückgewonnenen Embryo stammen.

4. Verfahren nach Anspruch 1. 2 oder 3, bei welchem die Empfänger-Eizelle aus Schritt (a) etwa 10 bis 14 Stunden nach der Ovulation entnommen wurde.

5. Verfahren nach Anspruch 4, bei welchem die Eizelle etwa 39 Stunden nach Verabreichung von chorialem Human-Gonadotropin oder 87 Stunden nach Verabreichung von Prostaglandin F₂α an die Spender-Kuh entnommen wurde.

6. Verfahren nach einem der vorangegangenen Ansprüche, wobei die Spenderzellen in Schritt (b) von einem Embryo stammen, der zur späteren Verwendung tiefgefroren und vor dieser Verwendung aufgetaut wurde.

7. Verfahren nach einem der vorangegangenen Ansprüche, wobei in Schritt (a) der Anteil des entfernten Eiplasmas der an das Polkörperchen angrenzende und dieses enthaltende ist.

8. Verfahren nach Anspruch 7, wobei in Schritt (a) der Anteil des entfernten Eiplasmas von einem Viertel bis zu einem Achtel des Gesamtvolumens der Zelle beträgt.

9. Verfahren nach einem der Ansprüche 1 bis 7, wobei in Schritt (a) etwa die Hälfte des Eiplasmas aus der Empfänger-Eizelle entfernt und in eine Fremdeihülle eingefügt wird, wobei diese Fremdeihülle zur Aufnahme des Eiplasmas durch Entfernung aus einer Eizelle vorbereitet worden war, und welches die Schritte (b) bis (d) unter Verwendung der Fremdeihülle mit dem eingefügten Eiplasma als Empfänger-Eizelle umfaßt.

10. Verfahren nach einem der vorangegangenen Ansprüche, wobei die Spenderzellen von einem Embryo einer befruchteten Spender-Kuh oder einer früheren Zellkerntransplantation, wie durch Schritte (a) bis (d) in Anspruch 1 definiert, stammen.

## Revendications (Revendications pour l'(les) Etat(s) contractant(s) suivant(s): AT, BE, IT, LU, SE)

1. Procédé de transplantation nucléaire *in vitro* comprenant les étapes suivantes consistant à :
(a) enlever une partie du cytoplasme d'un ovocyte receveur collecté à partir d'une vache donneuse;
(b) séparer en cellules donneuses uniques en vue d'une transplantation nucléaire les cellules d'un embryon bovin à un stade égal à ou supérieur au stade 32 cellules;
(c) insérer une cellule donneuse dans l'espace périvitellin de l'ovocyte receveur; et
(d) électrofuser le fragment d'ovocyte et la cellule donneuse pour former un embryon qui convient pour la culture dans le but de fournir un embryon transférable à une vache receveuse pour la gestation.

2. Procédé selon la revendication 1 dans lequel les cellules donneuses à l'étape (b) sont issues d'un embryon du stade 32 cellules au stade morula compacte.

3. Procédé selon la revendication 1 ou 2 dans lequel les cellules donneuses à l'étape (b) sont issues d'un embryon récupéré par entraînement de l'embryon hors de l'utérus d'une vache donneuse inséminée par un procédé non-chirurgical.

4. Procédé selon la revendication 1, 2 ou 3 dans lequel l'ovocyte receveur de l'étape (a) a été collecté environ 10 à 14 heures après l'ovulation.

5. Procédé selon la revendication 4 dans lequel l'ovocyte a été collecté environ 39 heures après administration de gonadotrophine chorionique humaine ou 87 heures après administration de prostaglandine F₂α à la vache donneuse.

6. Procédé selon l'une quelconque des revendications précédentes dans lequel les cellules donneuses à l'étape (b) sont issues d'un embryon qui a été congelé pour une utilisation ultérieure et qui est décongelé avant une telle utilisation.

7. Procédé selon l'une quelconque des revendications précédentes dans lequel à l'étape (a) la partie du cytoplasme enlevée est adjacente à et comprend le globule polaire.

8. Procédé selon la revendication 7 dans lequel à l'étape (a) la partie du cytoplasme enlevée représente de un quart à un huitième du volume total de la cellule.

9. Procédé selon l'une quelconque des revendications 1 à 7, dans lequel à l'étape (a) environ la moitié du cytoplasme est enlevé de l'ovocyte receveur et introduit dans une zone pellucide étrangère, ladite zone pellucide étrangère ayant été préparée à recevoir le cytoplasme par enlèvement d'un ovocyte, et qui comprend les étapes (b) à (d) en utilisant la zone pellucide étrangère avec le cytoplasme introduit comme ovocyte receveur.

10. Procédé selon l'une quelconque des revendications précédentes dans lequel les cellules donneuses sont issues d'un embryon issu d'une vache donneuse inséminée ou d'une transplantation nucléaire antérieure telle que définie par les étapes (a) à (d) de la revendication 1.

## Revendications (Revendications pour l'(les) Etat(s) contractant(s) suivant(s): DE, GB, NL, FR)

1. Procédé de transplantation nucléaire *in vitro* comprenant les étapes suivantes consistant à :
(a) enlever une partie du cytoplasme d'un ovocyte receveur collecté à partir d'une vache donneuse;
(b) séparer en cellules donneuses uniques en vue d'une transplantation nucléaire les cellules d'un embryon bovin à un stade supérieur au stade 32 cellules;
(c) insérer une cellule donneuse dans l'espace périvitellin de l'ovocyte receveur; et
(d) électrofuser le fragment d'ovocyte et la cellule donneuse pour former un embryon qui convient pour la culture dans le but de fournir un embryon transférable à une vache receveuse pour la gestation.

2. Procédé selon la revendication 1 dans lequel les cellules donneuses à l'étape (b) sont issues d'un embryon du stade 32 cellules au stade morula compacte.

3. Procédé selon la revendication 1 ou 2 dans lequel les cellules donneuses à l'étape (b) sont issues d'un embryon récupéré par entraînement de l'embryon hors de l'utérus d'une vache donneuse inséminée par un procédé non-chirurgical.

4. Procédé selon la revendication 1, 2 ou 3 dans lequel l'ovocyte receveur de l'étape (a) a été collecté environ 10 à 14 heures après l'ovulation.

5. Procédé selon la revendication 4 dans lequel l'ovocyte a été collecté environ 39 heures après administration de gonadotrophine chorionique humaine ou 87 heures après administration de prostaglandine F₂α à la vache donneuse.

6. Procédé selon l'une quelconque des revendications précédentes dans lequel les cellules donneuses à l'étape (b) sont issues d'un embryon qui a été congelé pour une utilisation ultérieure et qui est décongelé avant une telle utilisation.

7. Procédé selon l'une quelconque des revendications précédentes dans lequel à l'étape (a) la partie du cytoplasme enlevée est adjacente à et comprend le globule polaire.

8. Procédé selon la revendication 7 dans lequel à l'étape (a) la partie du cytoplasme enlevée représente de un quart à un huitième du volume total de la cellule.

9. Procédé selon l'une quelconque des revendications 1 à 7, dans lequel à l'étape (a) environ la moitié du cytoplasme est enlevé de l'ovocyte receveur et introduit dans une zone pellucide étrangère, ladite zone pellucide étrangère ayant été préparée à recevoir le cytoplasme par enlèvement d'un ovocyte, et qui comprend les étapes (b) à (d) en utilisant la zone pellucide étrangère avec le cytoplasme introduit comme ovocyte receveur.

10. Procédé selon l'une quelconque des revendications précédentes dans lequel les cellules donneuses sont issues d'un embryon issu d'une vache donneuse inséminée ou d'une transplantation nucléaire antérieure telle que définie par les étapes (a) à (d) de la revendication 1.
